# EUROPEAN PATENT APPLICATION

(11) **EP 3 906 793 A1**
(43) Date of publication of application: **10.11.2021**
(21) Application number: 20173181.7
(22) Date of filing: 06.05.2020
(51) Int. Cl.: A24F 40/49, A24F 40/50, A24F 40/60, A24F 40/65, A24F 40/53

(54) **A SMOKING SUBSTITUTE DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A smoking substitute device configured to generate an aerosol from an aerosol forming precursor. The device comprises: an ID device configured to read ID information identifying a person from an ID medium presented to the ID device by a user of the smoking substitute device; a biometric device configured to detect a biometric characteristic of the user. The smoking substitute device is configured to control operation of the smoking substitute device based on the ID information read by the ID device and based on the biometric characteristic detected by the biometric device

## Description

### TECHNICAL FIELD

The present invention relates to smoking substitute devices and also to a system including a smoking substitute device, a method performed by a smoking substitute device, a computer-readable medium configured to cause a smoking substitute device to perform a method, and electrical circuitry configured to cause a smoking substitute device to perform a method.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products. Some smoking substitute systems use smoking substitute articles (also referred to as a "consumables") that are designed to resemble a traditional cigarette and are cylindrical in form with a mouthpiece at one end.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach. A smoking substitute approach corresponds to the manner in which the substitute system operates for a user.

One approach for a smoking substitute device is the so-called "vaping" approach, in which a vapourisable liquid, typically referred to (and referred to herein) as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings. The base liquid may include propylene glycol and/or vegetable glycerin.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e-cigarette is an open system device which includes a main body, a (refillable) tank, and a mouthpiece. The main body and tank are physically and electrically coupled together by screwing one to the other. The mouthpiece and refillable tank are physically coupled together by screwing one of the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

There may be a need for improved design of smoking substitute systems, in particular HT smoking substitute systems, to enhance the user experience and improve the function of the HT smoking substitute system.

An example of the HT approach is the IQOS™ smoking substitute device from Philip Morris Ltd. The IQOS™ smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HT approach is the device known as "Glo"™ from British American Tobacco p.l.c. Glo™ comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier), such as vegetable glycerine ("VG") or propylene glycol ("PG").

The present inventor(s) have observed that most smoking substitute devices currently on the market are configured to operate in isolation of other devices, which limits the functions the smoking substitute devices can perform.

The present inventors have observed that there is a potential need to provide security in relation to smoking substitute devices, to ensure that they are not misused or used by non-authorised persons.

The present invention has been devised in light of the above considerations.

### Summary of the Invention

According to a first aspect, the present invention may provide a smoking substitute device configured to generate an aerosol from an aerosol forming precursor, the device comprising:
an ID device configured to read ID information identifying a person from an ID medium; a biometric device configured to detect a biometric characteristic of the user;
wherein the smoking substitute device is configured to control operation of the smoking substitute device based on the ID information read by the ID device and based on the biometric characteristic detected by the biometric device.

The ID medium may be presented to the ID device by a user of the smoking substitute device. The ID medium may be arranged in a consumable, which is presented to a body of the device on coupling of the consumable to the body or activation of the device body.

The ID medium may comprise, for example, a passport or a driving licence or any other suitable ID medium suitable for identifying a person. The smoking substitute device may be configured to restrict the permitted ID media to those which have been obtained from official government sources or from other legally verifiable sources.

The smoking substitute device may be configured to store ID information read by the ID device, preferably in encrypted form.

The smoking substitute device may be configured to store a biometric characteristic of the user detected by the biometric device, preferably in encrypted form.

The smoking substitute device may be configured to perform an ID authorisation check operation that includes:
operating the ID device to read the ID information from the ID medium;
determining whether the user is authorised to use the smoking substitute device based on an analysis of at least some of the ID information read by the ID device

The smoking substitute device may be configured to perform an ID authorisation check operation that includes:
operating the ID device to read the ID information from the ID medium, wherein the ID information read by the ID device includes a biometric characteristic of the person identified by the ID information;
operating the biometric device to detect a biometric characteristic of the user of the smoking substitute device;
determining whether the biometric characteristic detected by the biometric device is from the same person as the biometric characteristic included in the ID information based on a comparison of the biometric characteristic detected by the biometric device and the biometric characteristic included in the ID information; and
determining whether the user is authorised to use the smoking substitute device based on (i) whether it is determined that the biometric characteristic detected by the biometric device is from the same person as the biometric characteristic included in the ID information; and (ii) an analysis of at least some of the ID information read by the ID device.

For example, it may only be determined that the user is authorised to use the smoking substitute device if (i) it is determined that the biometric characteristic detected by the biometric device is from the same person as the biometric characteristic included in the ID information AND (ii) it is determined that the user is authorised to use the smoking substitute device based on analysis of at least some of the ID information read by the ID device (e.g. by passing an age verification test).

In some examples (e.g. of any ID authorisation check as defined above), determining whether the user is authorised to use the smoking substitute device based on an analysis of at least some of the ID information read by the ID device includes:
determining whether the person identified by the ID information passes an age verification test based on an analysis of at least some of the ID information read by the ID device.

The smoking substitute device may be configured to determine that the user is authorised to use the smoking substitute device if, as a result of an analysis of at least some of the ID information read by the ID device, it is determined that the person identified by the ID information passes the age verification test. The smoking substitute device may be configured to determine that the person identified by the ID information passes the age verification test if the analysis of the at least some of the ID information read by the ID device indicates that the person identified by the ID information is at least a predetermined minimum age (e.g. 18 years old).

The smoking substitute device may be configured to determine that the user is not authorised to use the smoking substitute device if, as a result of an analysis of at least some of the ID information read by the ID device, it is determined that the person identified by the ID information fails the age verification test. The smoking substitute device may be configured to determine that the person identified by the ID information fails the age verification test if the analysis of the at least some of the ID information read by the ID device indicates that the person identified by the ID information is younger than the predetermined minimum age (e.g. younger than 18 years old).

The smoking substitute device may be configured to, if it is determined from an ID authorisation check (e.g. any authorisation check as defined above) that the user is authorised to use the smoking substitute device:
store a biometric characteristic of the user detected by the biometric device, or a biometric characteristic included in the ID information read from the ID medium, as an authorised biometric characteristic.

An authorised biometric characteristic may be understood a biometric characteristic of a person for which a determination has been made that the person is authorised to use the smoking substitute device.

The stored authorised biometric characteristic may, for example, take the form of a biometric characteristic stored with metadata identifying the biometric characteristic as belonging to a person authorised to use the smoking substitute device.

The stored authorised biometric characteristic may, for example, take the form of a biometric characteristic stored in a memory location where only authorised biometric characteristics is permitted to be stored.

Preferably, the authorised biometric characteristic is stored in a memory of the smoking substitute device. This makes a subsequent biometric authorisation check (see below) more convenient.

However, in other examples, the authorised biometric characteristic may be stored in an external (e.g. peripheral) device configured to be connected (e.g. via a wired or wireless connection) to the smoking substitute device.

The smoking substitute device may be configured to perform a biometric authorisation check operation that includes:
operating the biometric device to detect a biometric characteristic of the user of the smoking substitute device;
determining whether the user is authorised to use the smoking substitute device based on a comparison of the biometric characteristic detected by the biometric device and a stored authorised biometric characteristic.

Preferably, the authorised biometric characteristic is stored in a memory of the smoking substitute device (e.g. as a consequence of the smoking substitute device determining from an ID authorisation check that the user is authorised to use the smoking substitute device - see above). This is makes the biometric authorisation check (see below) more convenient, as it doesn't involve an external (e.g. peripheral) device.

However, in other examples, the authorised biometric characteristic may be stored in an external (e.g. peripheral) device configured to be connected (e.g. via a wired or wireless connection) to the smoking substitute device.

The step of determining whether user is authorised to use the smoking substitute device based on a comparison of the biometric characteristic detected by the biometric device and the stored authorised biometric characteristic may involve:
determining whether the biometric characteristic detected by the biometric device is from the same person as the stored authorised biometric characteristic based on a comparison of the biometric characteristic detected by the biometric device and the stored authorised biometric characteristic; and
determining that the user is authorised to use the smoking substitute device if it is determined that the biometric characteristic detected by the biometric device is from the same person as the stored authorised biometric characteristic.

Determining whether a first biometric characteristic (e.g. the biometric characteristic detected by the biometric device) is from the same person as a second biometric characteristic (e.g. the stored authorised biometric characteristic) may involve determining a level of similarity between the two biometric characteristics (e.g. expressed as a probability) and, if the level of similarity is above a predetermined threshold, determining that the first and second biometric characteristics are from the same person. Such methods are well known in the field of biometrics and need not be described further herein.

The smoking substitute device may be configured to, based on the outcome of the biometric authorisation check:
permit the user to perform one or more operations with the smoking substitute device, if it is determined from the biometric authorisation check that the user is authorised to use the smoking substitute device;
prevent the user from performing the one or more operations with the smoking substitute device, if it is determined from the biometric authorisation check that the user is not authorised to use the smoking substitute device.

For avoidance of any doubt, the user need not be permanently prevented from performing the one or more operations if it is determined from the biometric authorisation check that the user is not authorised to use the smoking substitute device, since that user may be permitted to perform the one or more operations if it is determined from a subsequent biometric authorisation check that the user is authorised to use the smoking substitute device.

If it is determined from the biometric authorisation check that the user is authorised to use the smoking substitute device, the smoking substitute device may enter (or be maintained in) an unlocked mode, in which the smoking substitute device is permitted to perform the one or more operations (in this case, the one or more operations may be referred to as "unlocked mode" operations).

If it is determined from the biometric authorisation check that the user is not authorised to use the smoking substitute device, the smoking substitute device may enter (or be maintained in) a locked mode, in which the smoking substitute device is not permitted to perform the one or more operations.

The smoking substitute device may be configured to switch from a locked mode to the unlocked mode if it is determined from a biometric authorisation check that the user is authorised to use the smoking substitute device.

The one or more operations (permitted, if it is determined from the biometric authorisation check that the user is authorised to use the smoking substitute device) preferably include: activation of the smoking substitute device to produce vapour (i.e. a user is prevented from using the smoking substitute device to produce vapour if it is determined from the biometric authorisation check that the user is not authorised to use the smoking substitute device).

The one or more operations (permitted, if it is determined from the biometric authorisation check that the user is authorised to use the smoking substitute device) may include one or more of:
activation of the smoking substitute device to produce vapour;
the smoking substitute device wirelessly connecting to a mobile device;
transitioning the smoking substitute device to a new operating mode (e.g. a setup/administrator mode, or an unlocked mode).

The smoking substitute device may be configured to perform an ID authorisation check as part of a set-up (or registration or activation) process for the smoking substitute device. For example, the smoking substitute device may be configured to perform an ID authorisation check after a predetermined usage criteria relating to the smoking substitute device is met (e.g. after a predetermined number of activations or "puffs" have been performed using the smoking substitute device).

The smoking substitute device may be configured to perform a biometric authorisation check at randomly determined intervals.

The smoking substitute device may be configured to perform a biometric authorisation check according to a predetermined schedule.

The biometric device may be configured to detect a biometric characteristic that includes any one or more of: a fingerprint; a thumbprint; an iris, a retina or other eye-related characteristic; a facial characteristic; or DNA.

The ID device may be any device capable of reading ID information from an ID medium.

By way of non-limiting example, the ID device may be:
a reading device (e.g. an NFC or RFID reader) configured to read ID information (preferably wirelessly) from a memory included in/on the ID medium (e.g. the memory may be include in an NFC chip/tag or RFID chip/tag);
a camera configured to read ID information from an optical pattern (e.g. alphanumeric code, or other machine readable pattern such as a barcode or QR code) on the ID medium;
a magnetic code reader (e.g. a magnetic stripe reader) configured to read ID information from a magnetic pattern on an ID medium.

For avoidance of any doubt, the camera, if present, may be configured to detect light in any suitable wavelength band, e.g. visible light or IR light.

The smoking substitute device may be configured to store (in any location as defined previously) authorised biometric characteristics for multiple users who have been authorised to use the smoking substitute device. Thus, it may be possible for multiple users to be authorised to use the smoking substitute device. There may be a limit to the number of users that can be authorised to use the smoking substitute device.

A second aspect of the present invention may provide a system including:
a smoking substitute device according to the first aspect of the invention;
an ID medium containing ID information identifying a person;
wherein the ID device is configured to read ID information identifying the person from the ID medium if the ID medium is presented to the ID device by a user of the smoking substitute device.

The second aspect of the present invention may provide the ID device per se.

The system may include one or more external (e.g. peripheral) devices (e.g. a mobile device, e.g. an application server) configured to be in communication (e.g. wired or wireless communication, preferably wireless communication) with the smoking substitute device.

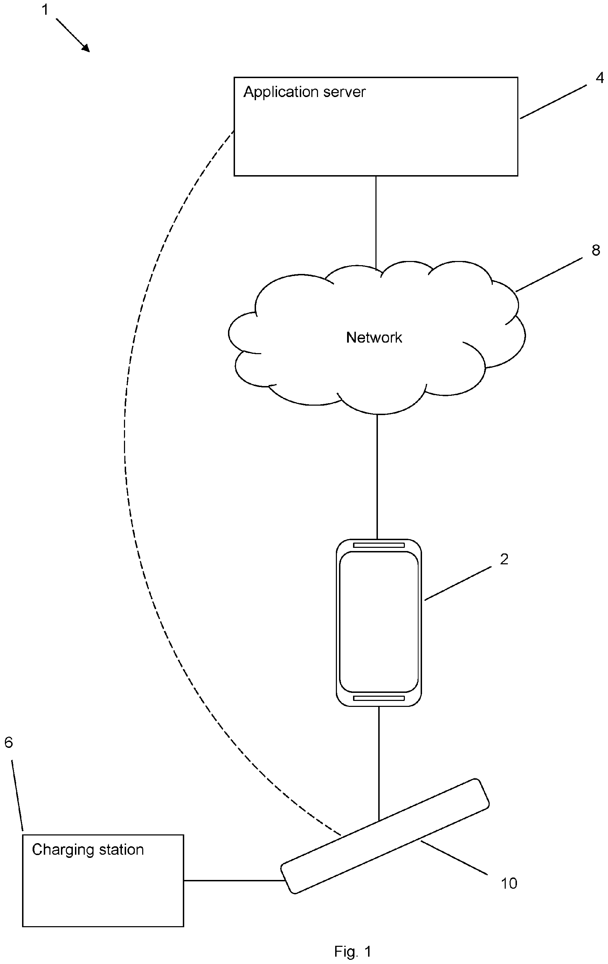

Example external devices are shown and discussed in relation to Fig. 1, for example.

A third aspect of the present invention may provide use of an ID medium containing ID information identifying a person containing for the smoking substitute device according to the first aspect of the invention.

A fourth aspect of the present invention may provide a method of operating a smoking substitute device, the method comprising: controlling an operation of the smoking substitute device based on ID information identifying a person from an ID medium and biometric information of a biometric characteristic of the person.

The fourth aspect of the invention may alternatively/additionally provide a method, performed by a smoking substitute device, the method comprising:
operating an ID device of the smoking substitute device to read ID information identifying a person from an ID medium presented to the ID device by a user of the smoking substitute device;
operating a biometric device of the smoking substitute device to detect a biometric characteristic of the user;
controlling operation of the smoking substitute device based on the ID information read by the ID device and based on the biometric characteristic detected by the biometric device.

The smoking substitute device may be as set out in relation to the first aspect of the invention.

A fifth aspect of the present invention may provide a computer readable medium comprising computer-readable instructions configured to cause a smoking substitute device to perform a method according to the fourth aspect of the present invention.

A sixth aspect of the present invention may provide electrical circuitry configured to cause a smoking substitute device to perform a method according to the fourth aspect of the invention.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### Summary of the Figures

Embodiments and experiments illustrating the principles of the invention will now be discussed with reference to the accompanying figures in which:
**Figure 1** shows an example system for managing a smoking substitute device.
**Figure 2(a)** shows an example smoking substitute device for use as the smoking substitute device in the system of Fig. 1.
**Figure 2(b)** shows the main body of the smoking substitute device of Fig. 2(a) without the consumable.
**Figure 2(c)** shows the consumable of the smoking substitute device of Fig. 2(a) without the main body.
**Figure 3(a)** is a schematic view of the main body of the smoking substitute device of Fig. 2(a).
**Figure 3(b)** is a schematic view of the consumable of the smoking substitute device of Fig. 2(a).
**Figure 4(a)** shows an example methods that may be implemented by the smoking substitute device of Fig. 2(a).
**Figure 4(b)** shows an example methods that may be implemented by the smoking substitute device of Fig. 2(a).
**Figure 5** shows an example methods that may be implemented by the smoking substitute device of Fig. 2(a).

### Detailed Description of the Invention

A smoking substitute device as described herein may be configured to generate an aerosol from an aerosol forming substrate. The smoking substitute device may be configured to deliver the aerosol to a user for inhalation. For avoidance of any doubt, the smoking substitute device could be either a vaping smoking substitute device, or a heat not burn smoking substitute device (such devices are described in the background section, above). As used herein, the term "aerosol" may include a suspension of aerosol forming substrate, included as one or more of: solid particles; liquid droplets; gas. Said suspension may be in a gas including air. The aerosol may include one or more components of the aerosol forming substrate. As used herein, the term "aerosol forming substrate" (also "aerosol-forming precursor" or "precursor") may refer to one or more of a: liquid; solid; gel; other substance (for which an aerosol is generated). An aerosol generating unit of the smoking substitute device may be configured to process the aerosol forming substrate to form an aerosol as defined herein. The term "aerosol" herein may be used interchangeably with the term "vapour".

Aspects and embodiments of the present invention will now be discussed with reference to the accompanying figures. Further aspects and embodiments will be apparent to those skilled in the art. All documents mentioned in this text are incorporated herein by reference.

Fig. 1 shows an example system 1 for managing a smoking substitute device 10.

The system 1 as shown in Fig. 1 includes a mobile device 2, an application server 4, an optional charging station 6, as well as the smoking substitute device 10.

The smoking substitute device 10 is configured to communicate wirelessly, e.g. via Bluetooth™, with an application (or "app") installed on the mobile device 2, e.g. via a suitable wireless interface (not shown) on the mobile device 2. The mobile device 2 may be a mobile phone, for example. The application on the mobile phone is configured to communicate with the application server 4, via a network 8. The application server 4 may utilise cloud storage, for example.

The network 8 may include a cellular network and/or the internet.

In other examples, the smoking substitute device 10 may be configured to communicate with the application server 4 via a connection that does not involve the mobile device 2, e.g. via a narrowband internet of things ("NB-loT") connection. In some examples, the mobile device 2 may be omitted from the system.

A skilled person would readily appreciate that the mobile device 2 may be configured to communicate via the network 8 according to various communication channels, preferably a wireless communication channel such as via a cellular network (e.g. according to a standard protocol, such as 3G or 4G) or via a WiFi network.

The app installed on the mobile device and the application server 4 may be configured to assist a user with their smoking substitute device 10, based on information communicated between the smoking substitute device 10 and the app and/or information communicated between the app and the application server 4.

The charging station 6 (if present) may be configured to charge (and optionally communicate with) the smoking substitute device 10, via a charging port on the smoking substitute device 10. The charging port on the smoking substitute device 10 may be a USB port, for example, which may allow the smoking substitute device to be charged by any USB-compatible device capable of delivering power to the smoking substitute device 10 via a suitable USB cable (in this case the USB-compatible device would be acting as the charging station 6). Alternatively, the charging station could be a docking station specifically configured to dock with the smoking substitute device 10 and charge the smoking substitute device 10 via the charging port on the smoking substitute device 10.

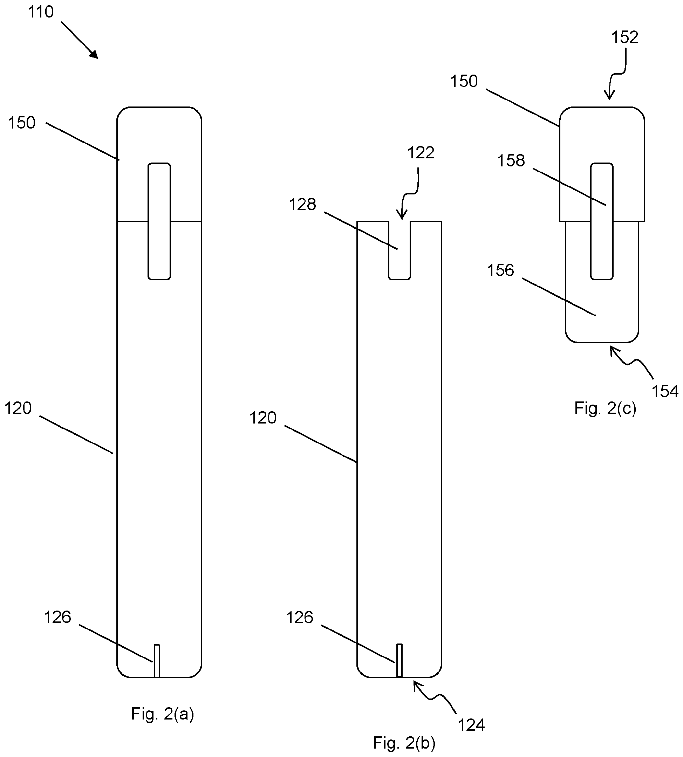

Fig. 2(a) shows an example smoking substitute device 110 for use as the smoking substitute device 10 in the system 1 of Fig. 1.

In this example, the smoking substitute device 110 includes a main body 120 and a consumable 150. The consumable 150 may alternatively be referred to as a "pod".

In this example, the smoking substitute device 110 is a closed system vaping device, wherein the consumable 150 includes a sealed tank 156 and is intended for one-use only.

Fig. 2(a) shows the smoking substitute device 110 with the main body 120 physically coupled to the consumable 150.

Fig. 2(b) shows the main body 120 of the smoking substitute device 110 without the consumable 150.

Fig. 2(c) shows the consumable 150 of the smoking substitute device 110 without the main body 120.

The main body 120 and the consumable 150 are configured to be physically coupled together, in this example by pushing the consumable 150 into an aperture in a top end 122 of the main body 120, e.g. with the consumable 150 being retained in the aperture via an interference fit. In other examples, the main body 120 and the consumable could be physically coupled together by screwing one onto the other, through a bayonet fitting, or through a snap engagement mechanism, for example. An optional light 126, e.g. an LED located behind a small translucent cover, is located a bottom end 124 of the main body 120. The light 126 may be configured to illuminate when the smoking substitute device 110 is activated.

The consumable 150 includes a mouthpiece (not shown) at a top end 152 of the consumable 150, as well as one or more air inlets (not shown in Fig. 2) so that air can be drawn into the smoking substitute device 110 when a user inhales through the mouthpiece. At a bottom end 154 of the consumable 150, there is located a tank 156 that contains e-liquid. The tank 156 may be a translucent body, for example.

The tank 156 preferably includes a window 158, so that the amount of e-liquid in the tank 156 can be visually assessed. The main body 120 includes a slot 128 so that the window 158 of the consumable 150 can be seen whilst the rest of the tank 156 is obscured from view when the consumable 150 is inserted into the aperture in the top end 122 of the main body 120.

In this present embodiment, the consumable 302 is a "single-use" consumable. That is, upon exhausting the e-liquid in the tank 156, the intention is that the user disposes of the whole consumable 150. In other embodiments, the e-liquid (i.e. aerosol former) may be the only part of the system that is truly "single-use". In such embodiments, the tank 156 may be refillable with e-liquid or the e-liquid may be stored in a non-consumable component of the system. For example, the e-liquid may be stored in a tank located in the device or stored in another component that is itself not single-use (e.g. a refillable tank).

The tank 156 may be referred to as a "clearomizer" if it includes a window 158, or a "cartomizer" if it does not.

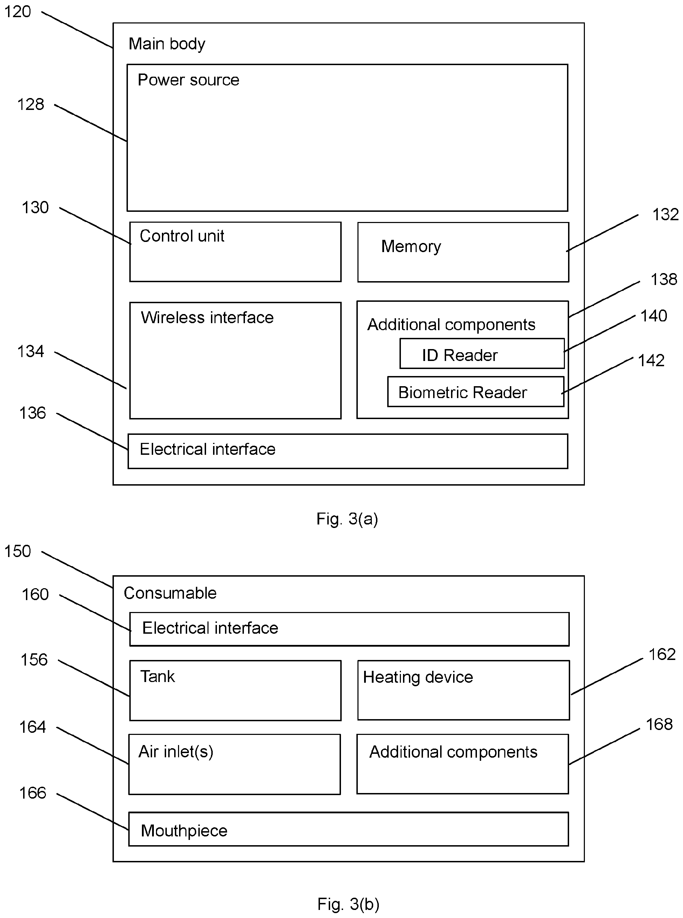

Fig. 3(a) is a schematic view of the main body 120 of the smoking substitute device 110.

Fig. 3(b) is a schematic view of the consumable 150 of the smoking substitute device 110.

As shown in Fig. 3(a), the main body 120 includes a power source 128, a control unit 130, a memory 132, a wireless interface 134, an electrical interface 136, and, optionally, one or more additional components 138.

The power source 128 is preferably a battery, more preferably a rechargeable battery.

The control unit 130 may include a microprocessor, for example.

The memory 132 is preferably includes non-volatile memory.

The wireless interface 134 is preferably configured to communicate wirelessly with the mobile device 2, e.g. via Bluetooth. To this end, the wireless interface 134 could include a Bluetooth™ antenna. Other wireless communication interfaces, e.g. WiFi, are also possible.

The electrical interface 136 of the main body 120 may include one or more electrical supply contacts. The electrical interface 136 may be located in, and preferably at the bottom of, the aperture in the top end 122 of the main body 120. When the main body 120 is physically coupled to the consumable 150, the electrical interface 136 may be configured to pass electrical power from the power source 128 to (e.g. a heating device of) the consumable 150 when the smoking substitute device 110 is activated, e.g. via the electrical interface 160 of the consumable 150 (discussed below). When the main body 120 is not physically coupled to the consumable 150, the electrical interface may be configured to receive power from the charging station 6.

The additional components 138 of the main body 120 may include the optional light 126 discussed above.

The additional components 138 of the main body 120 may, if the power source 128 is a rechargeable battery, include a charging port configured to receive power from the charging station 6. This may be located at the bottom end 124 of the main body 120. Alternatively, the electrical interface 136 discussed above is configured to act as a charging port configured to receive power from the charging station 6 such that a separate charging port is not required.

The additional components 138 of the main body 120 may, if the power source 128 is a rechargeable battery, include a battery charging control circuit, for controlling the charging of the rechargeable battery. However, a battery charging control circuit could equally be located in the charging station 6 (if present).

The additional components 138 of the main body 120 may include an airflow sensor for detecting airflow in the smoking substitute device 110, e.g. caused by a user inhaling through a mouthpiece 166 (discussed below) of the smoking substitute device 110. The smoking substitute device 110 may be configured to be activated when airflow is detected by the airflow sensor. This optional sensor could alternatively be included in the consumable 150 (though this is less preferred where the consumable 150 is intended to be disposed of after use, as in this example).

The additional components 138 of the main body 120 may include an actuator, e.g. a button. The smoking substitute device 110 may be configured to be activated when the actuator is actuated. This provides an alternative to the airflow sensor noted, as a mechanism for activating the smoking substitute device 110.

The additional components 138 of the main body 120 may include a reading device configured to read information associated with the consumable from a machine readable data source included in (e.g. contained in the body of, or attached to) the consumable 150.

In some examples, the reading device (if present) may be configured to read information from the machine readable data source non-wirelessly, e.g. using an electrical connection between the main body 120 and consumable 150.

For example, the reading device (if present) may include a set of one or more electrical communication contacts configured to read information from the machine readable data source via an electrical connection established between the set of one or more electrical communication contacts and the machine readable data source. Conveniently, the set of one or more electrical communication contacts of the reading device may be configured to provide the electrical connection by engaging with a set of one or more electrical communication contacts of the consumable 150, when the main body 120 and the consumable 150 are physically coupled together.

In some examples, the reading device (if present) may be configured to read information from the machine readable data source wirelessly, e.g. via electromagnetic waves or optically. Thus, for example, the machine readable data source included in the consumable 150 could be an RFID tag (in which case the reading device included in the main body 120 may be an RFID reader) or a visual data source such as a barcode (in which case the reading device included in the main body may be an optical reader, e.g. a barcode scanner). Various wireless technologies and protocols may be employed to allow the reading device to wirelessly read information from a machine readable data source included in or attached to the consumable 150, e.g. NFC, Bluetooth, Wi-Fi, as would be appreciated by a skilled person.

The reading device (if present) may be configured to write information associated with the consumable to the machine readable data source (e.g. wirelessly or non-wirelessly, via one of the mechanisms discussed above) in addition to being configured to read information associated with the consumable from the machine readable data source. In this case, the reading device may be referred to as a reading/writing device.

As shown in Fig. 3(b), the consumable 150 includes the tank 156, an electrical interface 160, a heating device 162, one or more air inlets 164, a mouthpiece 166, and, optionally, one or more additional components 168.

The electrical interface 160 of the consumable 150 may include one or more electrical supply contacts. The electrical interface 136 of the main body 120 and an electrical interface 160 of the consumable 150 are preferably configured to contact each other and therefore electrically couple the main body 120 to the consumable 150 when the main body 120 is physically coupled to the consumable 150. In this way, electrical energy (e.g. in the form of an electrical current) is able to be supplied from the power source 128 in the main body 120 to the heating device 162 in the consumable 150.

The heating device 162 is preferably configured to heat e-liquid contained in the tank 156, e.g. using electrical energy supplied from the power source 128. In one example, the heating device 162 may include a heating filament and a wick, wherein a first portion of the wick extends into the tank 156 in order to draw e-liquid out from the tank 156, and wherein the heating filament coils around a second portion of the wick located outside the tank 156. In this example, the heating filament is configured to heat up e-liquid drawn out of the tank 156 by the wick to produce an aerosol vapour.

The one or more air inlets 164 are preferably configured to allow air to be drawn into the smoking substitute device 110, when a user inhales through the mouthpiece 166.

The additional components 168 of the consumable 150 may include a machine readable data source, which may e.g. be contained in the body of, or attached to the consumable 150. The machine readable data source may store information associated with the consumable. The information associated with the consumable may include information concerning the content of the consumable (e.g. e-liquid type, batch number) and/or a unique identifier, for example.

The machine readable data source may be rewritable, e.g. a rewritable RFID chip, or read only, e.g. a visual data source such as a barcode. As indicated above, the additional components 138 of the main body 120 may include a reading device configured to read information associated with the consumable from the machine readable data source.

For example, the electrical interface 160 of the consumable 150 may include a set of one or more electrical communication contacts, which may allow a reading device of the main body to read information from a machine readable data source of the consumable, e.g. as discussed previously.

In use, a user activates the smoking substitute device 110, e.g. through actuating an actuator included in the main body 120 or by inhaling through the mouthpiece 166 as described above. Upon activation, the control unit 130 may supply electrical energy from the power source 128 to the heating device 162 (via electrical interfaces 136, 166), which may cause the heating device 162 to heat e-liquid drawn from the tank 156 to produce a vapour which is inhaled by a user through the mouthpiece 166.

Of course, a skilled reader would readily appreciate that the smoking substitute device 110 shown in Figs. 2 and 3 shows just one example implementation of a smoking substitute device, and that other forms of smoking substitute device could be used as the smoking substitute device 10 of Fig. 1.

By way of example, a HNB smoking substitute device including a main body and a consumable could be used as the smoking substitute device 10 of Fig. 1, instead of the smoking substitute device 110. One such HNB smoking substitute device is the IQOS™ smoking substitute device discussed above.

As another example, an open system vaping device which includes a main body, a refillable tank, and a mouthpiece could be used as the smoking substitute device 10 of Fig. 1, instead of the smoking substitute device 110. One such open system vaping device is the blu PRO™ e-cigarette discussed above.

As another example, an entirely disposable (one use) smoking substitute device could be used as the smoking substitute device 10 of Fig. 1, instead of the smoking substitute device 110.

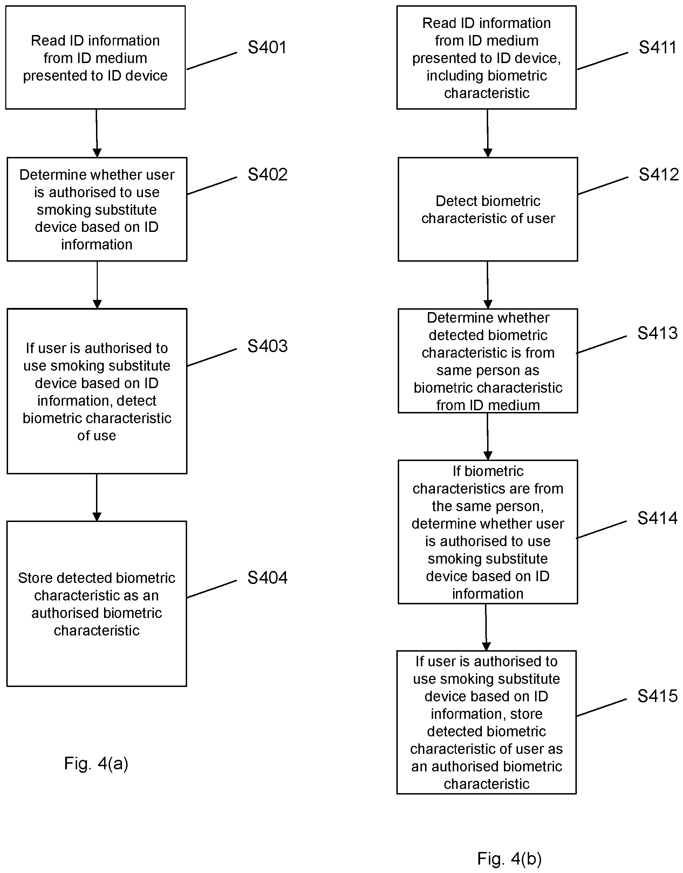

As can be seen from Fig. 4(a), the additional components 138 of the smoking substitute device 110 in Figure 3(a) include an ID device in the form of identification (ID) reader 140 and a biometric device in the form of biometric reader 142.

The terms "ID device" and "ID reader" may be used interchangeably in the discussions that follow.

The terms "biometric device" and "biometric reader" may be used interchangeably in the discussions that follow.

The ID reader 140 is configured to read ID information identifying a person from an ID medium presented to the ID device by a user of the smoking substitute device 110. The biometric reader 142 is configured to detect a biometric characteristic of the user.

The biometric reader 142 may be an electronic device configured to detect at least one biometric characteristics of the user (or prospective user) of the smoking substitute device 110, when he or she is in contact with, or in close proximity with, the smoking substitute device 110. The at least one biometric characteristic may include, by way of non-limiting example, identifiers associated with the user's person such as a fingerprint, a retina scan, an iris scan, DNA, a facial characteristic, image data. It may include a combination of multiple different types of such identifiers. The biometric reader142 may comprise or include a user-input component (not shown in Figure 3(a)) that is configured to receive input from the user, such as, by way of non-limiting example, a detector, scanner, sensor, camera, or a combination of such user-input components.

By way of example, the biometric reader 142 may comprise a fingerprint (or thumbprint) reader on a surface of the smoking substitute device 110, that the user can press his or her finger or thumb against. The biometric reader 142 may also have other functionality - for example, if it reads thumbprints, it may also be used as a button or actuator, depressible or otherwise actuatable by the user's thumb, for controlling one or more aspects of the smoking substitute device's functionality.

By way of example, the biometric reader 142 may comprise a camera that is configured to capture an image of some or all of the user's face. For example, it may capture an image or part or all of the user's eye or eyes.

The smoking substitute device 110 may comprise a screen or other output that is configured to provide the user with instructions, e.g. when and/or how to perform an ID authorisation check as described herein, e.g. when and/or how to perform a biometric authorisation check as described herein. Alternatively, or additionally, those instructions may be provided separately, e.g. via a mobile device wirelessly connected to the smoking substitute device 110. The smoking substitute device 110 may comprise an output (such as red/green lights or LED's) to inform the user when he or she is presenting his or her biometric data correctly, to the biometric data reader 142.

The biometric data reader 142 can include or be in connection with a controller or processor, e.g. control unit 130 that is configured to receive data representing a biometric characteristic from the user-input component(s) and to process it. For example, the controller or processor may be configured to compare the biometric characteristic detected by the biometric reader 142 and a stored authorised biometric characteristic, e.g. as described in more detail below.

The biometric reader 142 can include or be in connection with a memory for storing the biometric characteristic. For example, a biometric characteristic received via the biometric reader 142 during an initial set-up, activation or registration process may be stored in such a memory. The memory for storing a biometric characteristic detected by the biometric reader 142 may be part of the memory 132 of the smoking substitute device 110, shown in Figure 3(a) or it may be a separate memory (not shown). The memory for the biometric reader 142 may be configured to store a biometric characteristic detected by the biometric reader 142 in combination with, or linked to, user ID data. Alternatively, it may be configured only to store one or more biometric characteristics. In some arrangements, biometric characteristics might not be stored *per se* - but will instead only be used to verify that a user, whose biometric data is being detected, is authorised to use the smoking substitute device 110. The biometric characteristic may be stored in encrypted form, or in another secure form, such as in a password-protected memory. It may be possible to store biometric characteristics for multiple users on the smoking substitute device 110.

Of course, the biometric reader 142 may comprise a number of components or devices or sub-devices, which can work together for detecting one or more biometric characteristics, and/or two or more of which can detect different respective biometric characteristics of the user.

The ID reader 140 may comprise an electronic reading device that is configured to read ID information identifying a person from an electronically readable ID medium, when it is presented to, e.g. placed in close proximity with, the ID reader 140. The ID medium that the ID reader 140 is configured to read may comprise, by way of non-limiting example, a passport, driver's licence, student ID card, travel pass, national identity card and so on. The ID information that the ID 140 reader is configured to read may comprise or be associated with or linked to, one or more biometric characteristics of the person identified by the ID information, i.e. the person to whom the ID medium belongs. The ID information may also comprise user identifying or age-authenticating data such as, by way of non-limiting example, date of birth, national insurance number, national identification number, passport number and so on. The user-related data may be officially or legally verified or verifiable, for example it may comprise a date of birth obtained from a birth certificate, and/or age authentication data that has been obtained, or that can be or has been obtained, from an official and/or legal database.

The ID reader 140 may be configured to electronically obtain user-related data that is stored in, on or in association with the ID medium, using any suitable combination of hardware and software components. The ID reader 140 may comprise a number of components or devices or sub-devices, which can work together for reading ID information from an electronically readable ID medium, and/or two or more of which can perform different respective tasks.

The ID reader 140 may include or be in connection with a controller or processor, e.g. control unit 130, that is configured to receive the ID information from the ID reader 140 and to process it, e.g. as detailed further below. The ID reader 140 may also include or be in connection with a memory for storing the ID information. The memory for the ID information may be comprised within the memory 132 of the smoking substitute device 110, shown in Figure 3(a) or it may be a separate memory (not specifically shown).

As detailed further below, the ID reader 140 may be configured to wirelessly obtain ID information from an ID medium. The ID reader 140 may be configured to use any suitable electronic reading protocol. In some examples, the ID reader 140 may comprise a Near Field Communication (NFC) reader. In other examples, other types of ID readers may be used, e.g. RFID. For example, different respective types of ID reader may be appropriate for reading user-related data from different respective ID types - such as a passport and a driver's licence, respectively, for example. Or it may be that different types of ID reader are appropriate for use in different respective countries or jurisdictions, dependent on the prevailing types of ID media that are held by the inhabitants of each country or jurisdiction.

In the case of an NFC-enabled ID reader 140, as the skilled reader will be aware, NFC is a standard protocol for very short-range radio transmission. NFC is a 'proximity-based' technology, such that two NFC-enabled devices can communicate with one another, regardless of whether or not they are in line of sight of one another. Therefore, an NFC 'tag' (or chip, or circuit) can be incorporated physically within a user's ID medium, not necessarily visible from the outside, yet can still be readable by the NFC-enabled ID reader, once that tag is within a pre-determined short distance of the ID reader 140. For example, an NFC tag may be embedded within a page or cover or section of a user's passport or driver's licence. Alternatively, the NFC tag may be provided on a surface of the user's ID medium, for example in a 'sticker' form. Other types and locations of NFC tag are also possible.

NFC tends to have a relatively short physical range, usually limited to less than 20cm. However, having a relatively short range is generally not problematic for the arrangements discussed herein - and in fact may be regarded as beneficial - because the short range requires the ID medium and his or her smoking substitute device 110 to be physically very close to one another, in order for the ID reader 140 to read ID information from the ID medium. Therefore, the relatively short range reduces the risk of the wrong ID (or other entity comprising an NFC tag) being read, in any particular situation. This can help to reduce the risk of error, fraud, or misuse.

In the arrangement of Figure 3(a), the ID reader 140 is shown as being comprised within the main body 120 of the smoking substitute device 110. In some examples, it may comprise an 'active' NFC transceiver (or 'antenna') that is configured to be able to generate an RF (radiofrequency) field that can power a nearby 'passive' target such as an NFC tag comprised in, within or an ID medium presented to the ID reader 140. As the skilled reader will know, NFC uses inductive coupling between the active NFC transceiver and a passive target, in order for the transceiver to obtain data that is electronically stored on the target (i.e. on the NFC tag).

The ID reader 140 in Figure 3(a) may be configured for NFC communication with a passport, and potentially with other ID documents or sources. It is known for a passport (in the United Kingdom and elsewhere) to have an NFC tag embedded within one of the internal pages, or leaves, of the passport. Some passports have security measures such as foiled covers, which prevent the passport's NFC tag from being read when the passport is closed, but others do not. Other security measures may be in place, for example the NFC communication range for some passports may be very small, in order to ensure that a passport is not electronically read except when the holder expressly presents it to an NFC reader. The ID reader 140 may be configurable, either by the user and/or by the manufacturer or seller, to meet the relevant security standards for using NFC for reading the user's passport, wherein those security standards may vary according to jurisdiction/nationality.

The smoking substitute device 110, preferably the control unit 130 of the smoking substitute device 110, is configured to control operation of the smoking substitute device 110 based on the ID information read by the ID device 140 and based on the biometric characteristic detected by the biometric device 142.

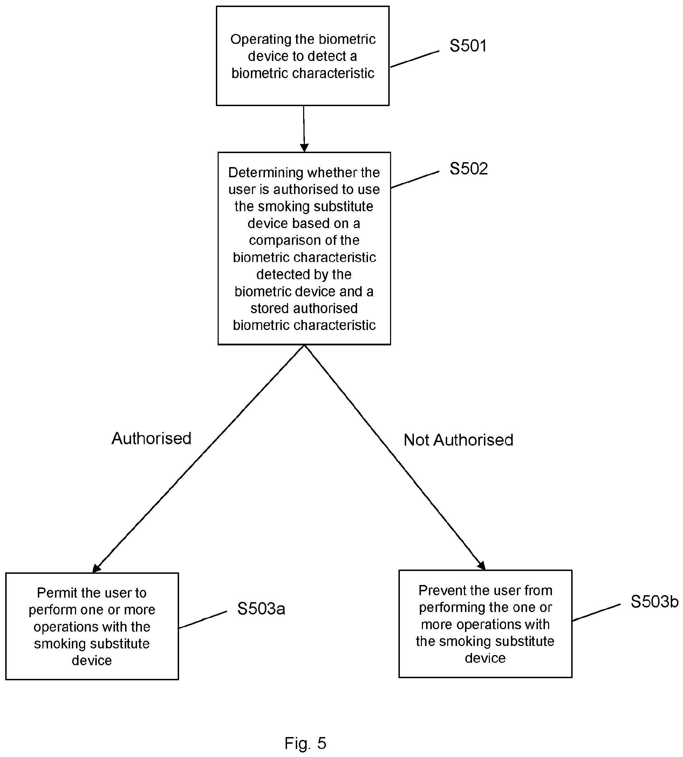

Figs. 4(a), 4(b) and 5, discussed below, show example methods in which operation of the smoking substitute device 110 may be controlled based on the ID information read by the ID device 140 and based on the biometric characteristic detected by the biometric device 140.

In the method of Fig. 4(a), the smoking substitute device 110 performs an ID authorisation check operation that includes:
operating the ID device 140 to read the ID information from an ID medium presented to the ID device 140 (S401);
determining whether the user is authorised to use the smoking substitute device based on the ID information read by the ID device 140 (S402);
if it determined from the ID authorisation check that the user is authorised to use the smoking substitute device 110, detecting a biometric characteristic of the user using the biometric device 142 (S403) and storing that biometric characteristic as an authorised biometric characteristic (S404).

It will be appreciated that the steps of Fig. 4(a) are exemplary and may be ordered differently. For example, the biometric characteristic may be obtained at an earlier stage of the method, but only stored as an authorised biometric characteristic if it is determined that the user is authorised to use the smoking substitute device 110.

In the method of Fig. 4(b), the smoking substitute device 110 performs an ID authorisation check operation that includes:
operating the ID device 140 to read the ID information from an ID medium presented to the ID device 140, wherein the ID information read by the ID device includes a biometric characteristic of the person identified by the ID information (S411);
operating the biometric device 142 to detect a biometric characteristic of the user of the smoking substitute device 110 (S412);
determining whether the biometric characteristic detected by the biometric device is from the same person as the biometric characteristic included in the ID information based on a comparison of the biometric characteristic detected by the biometric device and the biometric characteristic included in the ID information (S413);
if it is determined that the user is the same person as the person identified by the ID information, determining whether the user is authorised to use the smoking substitute device based on the ID information read by the ID device (S414);
if it determined based on the ID information read by the ID device that the user is authorised to use the smoking substitute device 110, storing the biometric characteristic detected by the biometric device as an authorised biometric characteristic (S415).

This is just one example of how the smoking substitute device 110 might make a determination of whether the user is authorised to use the smoking substitute device based on (i) whether it is determined that the biometric characteristic detected by the biometric device is from the same person as the biometric characteristic included in the ID information; and (ii) an analysis of at least some of the ID information read by the ID device. The steps of Fig. 4(b) could potentially be reordered to achieve the same effect, e.g. the analysis of the ID information could take place before the step of comparing the biometric characteristics. Preferably, however, the detected biometric characteristic is only stored as an authorised biometric characteristic if (i) it is determined that the biometric characteristic detected by the biometric device is from the same person as the biometric characteristic included in the ID information AND (ii) it is determined that the user is authorised to use the smoking substitute device based on analysis of at least some of the ID information read by the ID device (e.g. by passing an age verification test).

For avoidance of any doubt, the step of the smoking substitute device 110 determining whether the biometric characteristic detected by the biometric device is from the same person as the biometric characteristic included in the ID information may involve an external device, e.g. the application server 6. For example, in some examples, the smoking substitute device 110 could send the biometric characteristic detected by the biometric device, and the biometric characteristic included in the ID information, preferably in encrypted form, to the application server 6 (e.g. via the mobile device 2), with the application server 6 comparing the two biometric characteristics and sending the result of the comparison back to the smoking substitute device 110.

In some example variants of Fig. 4(b) (not shown), the biometric characteristic from the ID medium could be stored as the authorised biometric characteristic, although this might be is less preferred than what is shown in Fig. 4 (b) (storing the biometric characteristic detected by the biometric device 142 as the authorised biometric characteristic) since the biometric characteristic detected by the biometric device will in general be more similar to subsequent biometric characteristics detected by the biometric device, since the same detection device is being used.

A biometric characteristic detected by the biometric device 142, or otherwise obtained (e.g. from the ID medium - see above), may be stored locally, within a storage component or memory, such as a non-volatile memory, within the smoking substitute device 110, for example in the memory 132. For example, the memory 132 may comprise multiple compartments, configured for storing different respective types of data. For example, biometric characteristic detected by the biometric device 142, or otherwise obtained, may be stored separately to, say, device usage data. The memory or storage component may be configured to store the biometric characteristic(s) securely - for example this information may be encrypted, or password protected - in order to protect the biometric characteristic(s) in the event of loss or theft of the smoking substitute device 110, or in the event that the device 110 has multiple different users.

In either the method of Fig. 4(a) or Fig. 4(b), determining whether the user is authorised to use the smoking substitute device based on an analysis of at least some of the ID information read by the ID device preferably includes:
determining whether the person identified by the ID information passes an age verification test (e.g. is at least 18 years old) based on an analysis of at least some of the ID information read by the ID device.

In general, the ID information read from the ID medium would at least include a date of birth and/or age from the ID medium in order for an age verification test to be performed.

The age thresholds for the age verification process for a user's ID may be different, depending on which aerosol forming substrate the smoking substitute device 110 is configured to use. For example, there may be a minimum age limit of 16 years old for the user inhaling flavoured, nicotine-free vapours, using the smoking substitute device 110, whereas there may be a minimum age limit of 18 years old for the user inhaling nicotine-based vapours, using the smoking substitute device 110. The age threshold(s) may vary based on jurisdiction and/or based on the particular model or capabilities of the device 110.

The ID information read from the ID medium may be stored locally, within a storage component or memory, such as a non-volatile memory, within the smoking substitute device 110, for example in the memory 132. For example, the memory 132 may comprise multiple compartments, configured for storing different respective types of data. For example, the ID information read from the user's ID may be stored separately to, say, device usage data. The memory or storage component may be configured to store the ID information securely - for example this information may be encrypted, or password protected - in order to protect the ID information in the event of loss or theft of the smoking substitute device 110, or in the event that the device 110 has multiple different users.

In the method of Fig. 5, the smoking substitute device 110 performs a biometric authorisation check operation that includes:
operating the biometric device 142 to detect a biometric characteristic of the user of the smoking substitute device 110 (S501);
determining whether the user is authorised to use the smoking substitute device 110 based on a comparison of the biometric characteristic detected by the biometric device 142 and a stored authorised biometric characteristic (S502);
permit the user to perform one or more operations with the smoking substitute device, if it is determined from the biometric authorisation check that the user is authorised to use the smoking substitute device (S503a);
prevent the user from performing the one or more operations with the smoking substitute device, if it is determined from the biometric authorisation check that the user is not authorised to use the smoking substitute device (S503b).

The step of determining whether user is authorised to use the smoking substitute device based on a comparison of the biometric characteristic detected by the biometric device and the stored authorised biometric characteristic may involve:
determining whether the biometric characteristic detected by the biometric device is from the same person as the stored authorised biometric characteristic based on a comparison of the biometric characteristic detected by the biometric device and the stored authorised biometric characteristic; and
determining that the user is authorised to use the smoking substitute device if it is determined that the biometric characteristic detected by the biometric device is from the same person as the stored authorised biometric characteristic.

Determining whether a first biometric characteristic (e.g. the biometric characteristic detected by the biometric device) is from the same person as a second biometric characteristic (e.g. the stored authorised biometric characteristic) may involve determining a level of similarity between the two biometric characteristics (e.g. expressed as a probability) and, if the level of similarity is above a predetermined threshold, determining that the first and second biometric characteristics are from the same person. Such methods are well known in the field of biometrics and need not be described further herein.

For avoidance of any doubt, the step of the smoking substitute device 110 determining whether the user is authorised to use the smoking substitute device 110 based on a comparison of the biometric characteristic detected by the biometric device 142 and a stored authorised biometric characteristic may involve an external device, e.g. the application server 6. For example, in some examples, the smoking substitute device 110 could send the biometric characteristic detected by the biometric device 142, preferably in encrypted form, to the application server 6 (e.g. via the mobile device 2), with the application server 6 comparing the biometric characteristic detected by the biometric device 142 and an authorised biometric characteristic which is stored at the application server 6, and sending the result of the comparison back to the smoking substitute device 110. This variant may help avoid saving potentially sensitive biometric characteristics on the smoking substitute device 110 for any longer than is needed to complete the biometric authorisation check (for example, the smoking substitute device 110 may be configured to not store any biometric characteristics for any longer than a predetermined time period, which predetermined time period is preferably no more than 1 hour, or even no more than 5 minutes).

Preferably, the authorised biometric characteristic is stored in a memory of the smoking substitute device 110. This is makes the biometric authorisation check (see below) more convenient, as it doesn't require involvement an external device.

The stored authorised biometric characteristic may have been detected by the biometric device 142, e.g. as a consequence of an earlier ID authorisation check, such as exemplified by the method of Fig. 4(a) or Fig. 4(b). Or the stored authorised biometric characteristic may have originated from another source, e.g. from the ID medium, or from an external source (e.g. as noted above an authorised biometric characteristic may be stored in the application server 6).

However, in other examples, the authorised biometric characteristic may be stored in a peripheral device configured to be connected (e.g. via a wired or wireless connection) to the smoking substitute device, e.g. mobile device 2 or application server 6 as shown in Fig. 1.

As part of step S503a, the smoking substitute device 110 may enter (or be maintained in) an unlocked mode, in which the smoking substitute device is permitted to perform the one or more operations (in this case, the one or more operations may be referred to as "unlocked mode" operations).

As part of step S503b, the smoking substitute device 110 may enter (or be maintained in) a locked mode, in which the smoking substitute device is not permitted to perform the one or more "unlocked mode" operations.

The "unlocked mode" operations may include one or more of: activation of the smoking substitute device to produce vapour; the smoking substitute device wirelessly connecting to a mobile device; transitioning the smoking substitute device to a new operating mode (e.g. a setup/administrator mode, or an unlocked mode).

In some examples, the smoking substitute device 110 may be configured to allow one or more operations, even if it is determined that a user is not authorised to use the smoking substitute device based on a biometric authorisation check and/or ID authorisation check. For example, the smoking substitute device 110 may permit the device to be used with nicotine-free consumables even if it is determined that a user is not authorised to use the smoking substitute device based on a biometric authorisation check and/or ID authorisation check.

The smoking substitute device 110 may be configured to perform an ID authorisation check as part of a set-up (or registration or activation) process for the smoking substitute device.

In some arrangements, the smoking substitute device 110 may be configured so that a user will only be able to take a limited number of inhales (or 'puffs') from the smoking substitute device 110 before being required to 'register' or 'activate' the device 110, wherein the process of registering/activating includes performing an ID authorisation check, e.g. according to Fig. 4(a) or Fig. 4(b). In some arrangements, the user may be unable to factory reset the smoking substitute device before being authorised via an ID authorisation check (or a factory reset would not allow for additional usage before requiring the ID authorisation check). Such an arrangement can be provided in order to prevent the user from repeatedly factory resetting the device before the pre-determined number of inhales have been taken, thereby deliberately circumventing the requirement to register/activate his or her device. Such an arrangement can prevent deliberate misuse of the smoking substitute device 110, for example by underaged users or by other non-authorised users.

After an initial ID authorisation check, the smoking substitute device 110 may be configured to run one or more subsequent biometric authorisation checks, to check that the device 110 has not been passed on to a non-authorised user. For example, the smoking substitute device may be configured to perform, at randomly determined intervals or according to a predetermined schedule, one or more subsequent biometric authorisation checks.

In some examples, the smoking substitute device 110 or mobile device 2 connected to the smoking substitute device may be configured to notify the user of when an ID authorisation check and/or biometric check is required.

The smoking substitute device may be configured to store (in any location as defined previously) authorised biometric characteristics for multiple users who have been authorised to use the smoking substitute device. Thus, it may be possible for multiple users to be authorised to use the smoking substitute device. There may be a limit to the number of users that can be authorised to use the smoking substitute device.

The ID information may be read, for example, from an NFC tag on a passport or other ID medium. For example, the NFC tag on the passport may comprise a biometric characteristic such as fingerprint data, retina, iris or other eye-related data, or DNA-related data for the user. The NFC tag may also or instead comprise image data such as a photograph of the user's face, which can be read by the ID reader 140.

In some examples, other than in cases where an external device is specifically described as being involved, one or more of the above-described processes may be carried out only by the smoking substitute device 110, i.e. without involvement of other elements in a wider system such as a mobile device 2, an application server 6 and so on. In this way, these processes can be conducted without needing the smoking substitute device 110 to be connected to any other devices.

However, in some examples, external devices may be involved in one or more of the above described processes, and indeed some cases involving external devices have been described above.

The ID reader 140 and/or the biometric data reader 142 may also have additional functionality. For example, if the ID reader 140 is an NFC reader as described above, it may also be configured to use NFC to read data from a consumable 150 that comprises an NFC tag. For example, if the biometric data reader 142 is a camera, it may be used as a general-purpose camera, with captured photographs being transferrable to the mobile device 2 or other device using a suitable wired or wireless protocol.

The biometric data reader 142 may be configured to provide enhanced security for powering the smoking substitute device 110 on and off, or at least for enabling its e-liquid heating functionality. For example, the biometric data reader 142 may be a thumbprint reader and the user may have to present his or her thumb to activate the device 110 for every use.

In the arrangement of Figure 3(a), the biometric data reader 142 and the ID reader 140 are integrated within the main body 120 of the smoking substitute device 110. However, in other arrangements the biometric data reader 142 and/or the ID reader 140 may be detachable form the main body 120. The biometric data reader 142 and/or the ID reader 140 may thus be provided as one or more 'add-ons', which may be retrofitted to an existing smoking substitute device or added to a future smoking substitute device. For example, a smoking substitute device may have an integrated biometric data reader 142 and a detachable ID reader 140, since the ID reader may only be needed for occasional use for registering/age-verifying a new user.

The biometric data reader 142 may be configured to provide enhanced security for powering the smoking substitute device 110 on and off, or at least for enabling its e-liquid heating functionality. For example, the biometric data reader 142 may be a thumbprint reader and the user may have to present his or her thumb to activate the device 110 for every use.

In the arrangement of Figure 4(a), the biometric data reader 142 and the ID reader 140 are integrated within the main body 120 of the smoking substitute device 110. However, in other arrangements the biometric data reader 142 and/or the ID reader 140 may be detachable form the main body 120. The biometric data reader 142 and/or the ID reader 140 may thus be provided as one or more 'add-ons', which may be retrofitted to an existing smoking substitute device or added to a future smoking substitute device. For example, a smoking substitute device may have an integrated biometric data reader 142 and a detachable ID reader 140, since the ID reader may only be needed for occasional use for registering/age-verifying a new user.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A smoking substitute device configured to generate an aerosol from an aerosol forming precursor, the device comprising:
an ID device configured to read ID information identifying a person from an ID medium;
a biometric device configured to detect a biometric characteristic of the user;
wherein the smoking substitute device is configured to control operation of the smoking substitute device based on the ID information read by the ID device and based on the biometric characteristic detected by the biometric device.

2. The smoking substitute device of claim 1, wherein the smoking substitute device is configured to perform an ID authorisation check operation that includes:
operating the ID device to read the ID information from the ID medium;
determining whether the user is authorised to use the smoking substitute device based on an analysis of at least some of the ID information read by the ID device.

3. The smoking substitute device of any previous claim, wherein the smoking substitute device is configured to perform an ID authorisation check operation that includes:
operating the ID device to read the ID information from the ID medium, wherein the ID information read by the ID device includes a biometric characteristic of the person identified by the ID information;
operating the biometric device to detect a biometric characteristic of the user of the smoking substitute device;
determining whether the biometric characteristic detected by the biometric device is from the same person as the biometric characteristic included in the ID information based on a comparison of the biometric characteristic detected by the biometric device and the biometric characteristic included in the ID information; and
determining whether the user is authorised to use the smoking substitute device based on (i) whether it is determined that the biometric characteristic detected by the biometric device is from the same person as the biometric characteristic included in the ID information; and (ii) an analysis of at least some of the ID information read by the ID device.

4. The smoking substitute device of claim 2 or 3, wherein determining whether the user is authorised to use the smoking substitute device based on an analysis of the ID information read by the ID device includes:
determining whether the person identified by the ID information passes an age verification test based on an analysis of at least some of the ID information read by the ID device.

5. The smoking substitute device of any previous claim, wherein the smoking substitute device is configured to, if it is determined, from an authorisation check, that the user is authorised to use the smoking substitute device:
store a biometric characteristic of the user detected by the biometric device, or a biometric characteristic included in the ID information read from the ID medium, as an authorised biometric characteristic.

6. The smoking substitute device of any previous claim, wherein the smoking substitute device is configured to perform a biometric authorisation check operation that includes:
operating the biometric device to detect a biometric characteristic of the user of the smoking substitute device;
determining whether the user is authorised to use the smoking substitute device based on a comparison of the biometric characteristic detected by the biometric device and a stored authorised biometric characteristic.

7. The smoking substitute device of claim 6, wherein the smoking substitute device is configured to, based on the outcome of the biometric authorisation check:
permit the user to perform one or more operations with the smoking substitute device, if it is determined from the biometric authorisation check that the user is authorised to use the smoking substitute device;
prevent the user from performing the one or more operations with the smoking substitute device, if it is determined from the biometric authorisation check that the user is not authorised to use the smoking substitute device.

8. The smoking substitute device of claim 7, wherein the one or more operations include activation of the smoking substitute device to produce vapour.

9. The smoking substitute device of claim 7 or 8, wherein the smoking substitute device is configured to perform biometric authorisation check at randomly determined intervals, or according to a predetermined schedule.

10. The smoking substitute device of any preceding claim wherein the biometric device is configured to detect a biometric characteristic that includes any one or more of: fingerprint; thumbprint; iris, retina or other eye-related characteristic; a facial characteristic; or DNA.

11. The smoking substitute device of any preceding claim wherein the ID device includes:
a reading device configured to read ID information from a memory included in/on the ID medium;
a camera configured to read ID information from an optical pattern on the ID medium;
a magnetic code reader configured to read ID information from a magnetic pattern on an ID medium.

12. A system including:
the smoking substitute device according to any preceding claim;
an ID medium containing ID information identifying a person;
wherein the ID device is configured to read ID information identifying the person from the ID medium if the ID medium is presented to the ID device by a user of the smoking substitute device.

13. Use of an ID medium containing ID information identifying a person containing for the smoking substitute device of any of claims 1 to 11.

14. A method of operating a smoking substitute device, the method comprising:
controlling an operation of the smoking substitute device based on ID information identifying a person from an ID medium and biometric information of a biometric characteristic of the person.

15. A computer readable medium comprising computer-readable instructions configured to cause a smoking substitute device to perform a method according to claim 14.
